(19) Europäisches Patentamt
European Patent Office
Office européen des brevets

(11) Publication number: **0 055 474**
**B1**

(12) **EUROPEAN PATENT SPECIFICATION**

(45) Date of publication of patent specification: **30.04.86**

(21) Application number: **81110749.9**

(22) Date of filing: **23.12.81**

(51) Int. Cl.[4]: **A 01 N 41/10**, C 08 K 5/41, C 07 C 147/06

(54) **The use of beta-sulfonylacrylic acid derivatives on a dry blend containing them or pellets made therefrom in synthetic polymers.**

(30) Priority: **29.12.80 US 221119**
**29.12.80 US 221120**

(43) Date of publication of application:
**07.07.82 Bulletin 82/27**

(45) Publication of the grant of the patent:
**30.04.86 Bulletin 86/18**

(84) Designated Contracting States:
**AT BE CH DE FR GB IT LI NL SE**

(56) References cited:
**CH-A- 482 395**
**US-A-3 159 532**

**CENTRAL PATENT INDEX, Basic Abstracts Journal, Section C, Agdoc, Week X40, 1976, Nr. 74895x DERWENT PUBLICATIONS, LONDEN (GB)**

**The file contains technical information submitted after the application was filed and not included in this specification**

(73) Proprietor: **STAUFFER CHEMICAL COMPANY**
**Westport Connecticut 06880 (US)**

(72) Inventor: **Liu, Sophia Y.**
**264 Segovia Place**
**Fremont, Cal. 94538 (US)**
Inventor: **Gutmann, Arnold D.**
**202 The Crossways**
**Berkeley, Cal. 94708 (US)**
Inventor: **Williams, John Wesley**
**1397 Hale Street**
**Vallejo Cal. (US)**

(74) Representative: **Jaeger, Klaus, Dr. et al**
**JAEGER & PARTNER Patentanwälte Bergstrasse 48 1/2**
**D-8035 München-Gauting (DE)**

(56) References cited:
**CHEMICAL ABSTRACTS, volume 87, Nr. 25, December 19, 1977, page 80, ref. 200.987k COLUMBUS, OHIO (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 055 474 B1**

**Description**

This invention relates to the protection of synthetic polymeric materials against attack by microorganisms.

Synthetic, film-forming materials such as those used in the manufacture of plastic or polymeric films, and various types of plastics or polymers, are known to be subject to attack by microorganisms. Such microorganisms include bacteria, fungi and actinomycetes. The last mentioned are microorganisms found in soil which contain no chlorophyll. They are usually classified with the bacteria, but resemble both bacteria and fungi; they are intermediate in size between fungi and bacteria.

Such microorganisms attack plastics and polymers and can cause damage or deterioration ranging from discoloration and staining to embrittlement or actual disintegration, depending on the type of plastic or polymer, and the environment in which it is situated. Severe damage can be produced to plastics or polymers which are utilized in certain environments, such as those with high humidity. Plastics and polymers are commonly used for instance, in underground construction, in pipes and conduits, cables, sheathing and insulation. In such an environment, they are subject to severe deterioration by soil-borne microorganisms. Similarly, plastics and polymers used in materials such as swimming pool liners, awnings, camping equipment, and other articles for outdoor use, and in upholstery, car tops, shoes, boots and clothing, in which they may be exposed to natural humidity and/or sweat, possibly in combination with somewhat elevated temperatures, are subject to microbial deterioration.

In addition to physical deterioration of plastics and polymers, microorganisms growing on the surface of such materials can cause discoloration and/or staining thereof resulting in a shortening of the useful life of said materials for at least aesthetic purposes. Actinomycetes, in particular, growing on the surfaces of plastics and polymers can produce colored byproduct dyes which are soluble in the plasticizers used in such substances, and which migrate through the substance via the plasticizer, resulting in the phenomenon known as "pink staining." In addition, surface growth of microorganisms on polymers may interfere with functional performance, for instance when used as lubricating surfaces.

In order to prevent attack and deterioration or discoloration of polymeric or plastic materials by microorganisms, a number of compounds have been used as industrial biocides.

For use as a biocide in connection with plastics and polymers, a compound must have the following properties:

effectiveness at low levels against many microorganisms;

compatibility with plasticizers and other chemicals used in the formulation of plastic and polymeric products;

lack of a disadvantageous color or odor;

resistance to leaching from plastic or polymeric materials;

and particularly;

a) thermal stability at temperatures over 300°F. (148°C.) so that deterioration of the biocide does not occur during the processing of plastics and polymers;

b) mobility — the ability to migrate through the plastic, polymer or plasticizer utilized therein, so as to be dissipated therethrough; and

c) stability towards ultraviolet and other light radiation.

Such properties are necessary for plastics and polymers which are cast, rolled, molded, extruded, or otherwise fabricated into a continuous form, for use in various ways such as raw materials for the manufacture of plastic or polymer articles or as plastic or polymeric coatings, as well as plastics and polymers which are knitted or woven into continuous fibers.

Many of the industrial biocides currently used in connection with plastics and polymers are organometallics. These compounds are effective in preventing microbial attack on such materials. However, such compounds may be suspect for reasons of toxicity or environmental effect and problems caused by their handling and are now less accepted in some of the industrial uses in which they have hitherto been employed.

C.A. 87:200987k discloses a number of β-sulfonylacrylic acid derivatives and a synthesis for making such β-sulfonylacrylic acid derivatives. Derwent 74895X-40 discloses dichlorophenyl-sulfonyl-acrylonitrile as a non-medical fungicide being effective for controlling diseases of fruit trees and vegetables, in water such as industrial cooling water or water for paper making, in lubricant oil, pulp or aqueous emulsions. Finally, such arylsulfonyl alkenenitriles also are disclosed in US—A—3 159 532 as inhibiting the growth of microorganisms such as bacteria and fungi, and to provide especially a new method of treating soil to prevent substantial fungicidal damage to germination of seeds and the growth of plants in the soil. In consistency with their nature as acrylic acid derivatives both the latter documents disclose the β-sulfonylacrylic acid derivatives as a microbiocide for mainly liquid phase applications at room temperature.

In brief, this invention thus comprises a method of protecting a synthetic polymer composition, fabricated into a continuous form, comprising incorporating into such composition a microbiocidally effective amount of a compound having the formula

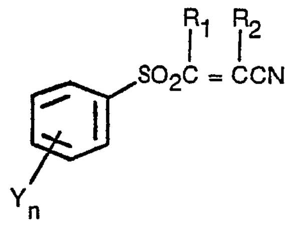

in which Y is hydrogen, $C_1$—$C_4$ alkyl, $C_1$—$C_4$ alkoxy, or halogen; $R_1$ and $R_2$ are independently hydrogen or methyl; and n is 1 or 2.

The polymers include polymeric materials which can be cast, extruded, injection molded, or compression molded into a desired state.

Certain polymers such as alkyd resins, polyester based urethanes, polyesters, and certain cellulosic polymers such as benzyl-, methyl-, hydroxyethyl-, and sodium carboxymethyl-cellulose, are susceptible in and of themselves to attack by microorganisms. Other polymers such as polyethylene and polystyrene may become susceptible after degradation through prolonged weathering. Still other plastics and polymers, such as polyvinyl chloride, are considered in and of themselves to be generally resistant to such attack. However, many substances utilized with polymers to produce polymeric or plastic products, for instance, flexible polyvinyl chloride sheeting, contain additives such as plasticizers, stabilizers, fillers, lubricants, thickening agents or starch sizings for synthetic fibers, which are susceptible to attack by microorganisms. Thus, plastics or other materials formulated from a polymer resistant to such attack but incorporating a substance such as a plasticizer, which is susceptible to such attack, are thereby rendered susceptible to deterioration, discoloration, and other damage from microorganisms. Alternatively, microorganisms can grow on debris, lubricants, or other materials adhering to the surface of a non-susceptible plastic or polymer. Metabolic products of such organisms may cause etching of the substrate material.

The compounds which have been found to be biocides for use with synthetic polymers are those having the formula

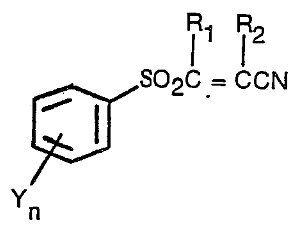

in which Y is hydrogen, $C_1$—$C_4$ alkyl, $C_1$—$C_4$ alkoxy, or halogen; $R_1$ and $R_2$ are independently hydrogen or methyl; and n is 1 or 2. Preferably the group Y is located para to the sulfonyl moiety. The term "halogen" includes chloro, bromo, iodo and fluoro, and preferably indicates chloro, bromo, or fluoro. The most preferred compound is that in which Y is p-methyl. Other preferred compounds are those in which Y is p-chloro, p-fluoro, p-methoxy or hydrogen. These compounds, except for those in which Y is alkoxy, are disclosed in U.S. Patents 3,159,532 and 3,159,666 of Heininger, et al., as part of a series of aryl sulfonyl alkene nitriles. As shown in those patents, these compounds are effective microbiocides for use in agricultural environments, particularly as soil microbiocides, and showed activity in vitro against a number of fungi and bacteria. Also mentioned with respect to the p-methyl compound is soap bacteriostatic and paint preservative activity. No mention is made in these patents of any utility with respect to synthetic polymers.

Fur use as an agricultural biocide, compounds must possess a necessary short term, ephemeral activity, as agricultural biocides must not leave residues in either the soil or the plants which have been treated. The properties necessary for use as a synthetic polymer biocide are different, however, from those required for effectiveness of an agricultural biocide. As pointed out above, particularly for use as plastics or polymer biocides, compounds must possess high thermal stability, ultraviolet radiation stability, and the ability to migrate through the plastic. Such properties are not necessarily possessed by compounds which are soil fungicides, nor are such properties required of, or necessarily possessed by compounds which are used as biocides for soap and for preservation of paint.

The compounds to which the present invention relates have been found to possess surprisingly high thermal stability, with the p-methyl compound being thermally stable at 250°C. They have been found to possess the necessary, unexpected ability to migrate through plastics, and the requisite, surprisingly high stability to ultraviolet radiation.

The compounds which are known may be prepared according to any of several processes described in the literature, for instance in U.S. Patents 3,159,666 and 3,541,119. The alkoxy-substituted compounds may be prepared by similar processes or by a method which comprises reacting an alkali metal (preferably sodium) salt of a sulfinic acid with 2-chloroacrylonitrile or an analog:

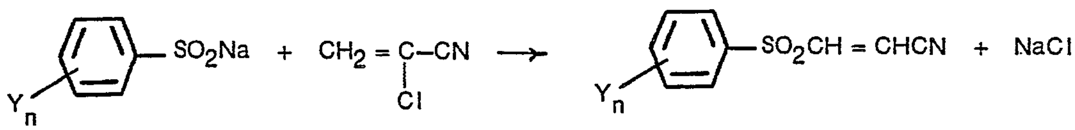

This reaction is generally conducted in aqueous media in the presence of buffers such as sodium acetate and/or boric acid, at approximately room temperature.

The following is an example of the preparation of one of the novel compounds by such technique.

Example 1
Preparation of 3-(4-methoxyphenylsulfonyl)acrylonitrile
(Compound 7 herein)

In a flask, 59.5 g (0.31 mol) sodium 4-methoxybenzenesulfinate, 25.4 g (0.31 mol) sodium acetate and 19.2 g (0.31 mol) boric acid were dissolved in a mixture of 150 ml water and 275 ml isopropyl alcohol. There was then added, with stirring, dropwise, 33.9 g (31 ml, 0.39 mol) 2-chloroacrylonitrile. The mixture was stirred for some time at room temperature. Water was then added and the mixture stirred for one hour. A precipitate formed, which was filtered, washed with water and ethanol, and dried.

The structure of the compound, which had a melting point of 120—122°C, was confirmed by infrared, nuclear magnetic resonance, and mass spectroscopy.

Tests were performed on a number of compounds falling within the generic class defined by the formula given above. These compounds are described in Table 1.

TABLE 1

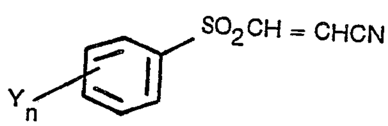

| Compound No. | $Y_n$ |
|---|---|
| 1 | 4-$CH_3$ |
| 2 | 4-Cl |
| 3 | 4-sec.$C_4H_9$ |
| 4 | 4-F |
| 5 | H |
| 6 | 2,5-Cl |
| 7 | 4-$OCH_3$ |

To determine the suitability of the compounds as biocides for these materials, tests were performed as described below.

A) Thermal Stability

Compound 1 was tested for thermal stability using a Perkin-Elmer DSC-2 differential scanning calorimeter. Samples of solid compound weighing between 5 and 20 mg were sealed into stainless steel pans and heated at a rate of 5.0°C/min from 40°C to 250°C. Thermograms of the compound's physical state were plotted against change in temperature. All showed approximately the same properties: a small endothermic transition occurring at about 75°C and melting at between about 125—135°C. Over the entire range tested, up to 250°C, there was no evidence of any unusual thermal instability.

B. Microbial Resistance Screening Test (plastics/polymers)

Compound 1 was utilized in this test. A dry powdered blend of polyvinyl chloride was prepared by dry mixing of the following ingredients (by weight): 100 parts Dia 450® polyvinyl chloride resin (Diamond Shamrock Chemical Corporation), 40 parts dioctyl phthalate plasticizer, 3.50 parts Mark KCB® cadmium-barium-zinc heat stabilizer/lubricant, 1.50 parts Mark C® organic phosphite heat stabilizer/lubricant (Mark chemicals available from Argus Chemical Division, Witco Chemical Co.), 0.25 parts stearic acid and 7.6 parts expoxidized soybean oil (plasticizer/stabilizer). This composition was then fluxed on hot rollers at 160°C (320°F) until homogeneously blended. Then a sufficient quantity of solid Compound 1 was added, to

provide a composition containing 1.0% weight of this compound. The composition was further milled for ten minutes, at this temperature, then sheeted off as a film.

Samples of the film thus prepared were submitted to 100 h of exposure treatment, in a xenon arc-type weatherometer (weather-simulating exposure test apparatus) programmed for continuous light with 18 min of water spray every two hours. The exposure was conducted according to ASTM Standard G26-70.

Prior to exposure, and after 100 h of exposure, samples of the films containing the test compound were tested for antimicrobial effect by the following procedure: The samples were placed on a nutrient agar inoculated with the test organism. Those samples inoculated with bacteria or actinomycetes were incubated for 24 h at 37°C, samples inoculated with fungi were incubated 14 d at 28°C. After the incubation period, the anti-microbial activity was evaluated by measuring (in millimeters) the size of the clear zone of no growth around the sample, and rating the degree of growth or stain visually.

The organisms utilized in the tests were:

*BACTERIA*
*Staphylococcus aureus*
*Klebsiella pneumoniae*
*Pseudomonas aeruginosa*
*Bacillus subtilus*

*ACTINOMYCETES*
*Stv. recticulum*
(pink staining organism)

FUNGI: A mixed fungal spore suspension of:
*Aspergillus niger*
*Aspergillus flavus*
*Penicillium funicolosum*
*Chaetomium globosum* ATCC 6205

The results of these tests are shown in the following Table II.

TABLE II

(ZONE OF INHIBITION, MM/GROWTH OR STAIN IN CONTACT AREA)

| | Staph. aureus | K. pneum. | Pseud. aer. | Bac. Subt. | Pink Stain | Mixed Fungi |
|---|---|---|---|---|---|---|
| Unexposed | 10/N | 4/N | 0/G | 10/N | 7/NS | 1.2/N |
| 100 hrs. exposure | 9/N | 3/N | 0/G | 9/G | 6/NS | 0/N |

N — no growth in contact area
G — growth in contact area
NS — no staining

C). Anti-microbial Screening Tests with Ultraviolet and Weather Exposure (plastics/polymers)

Dry blends of polyvinyl chloride were prepared as in the previous tests with the exception that no expodized soybean oil was included. To one set of blends there was additionally added one part Mark 202A® benzophenone ultraviolet light stabilizer. Thus, one set of blends contained an ultraviolet stabilizer, while the other set of blends was not stabilized against ultraviolet radiation.

The compounds in Table I above were incorporated into ultraviolet-stabilized and unstabilized polymer blends at levels of 0.5 and 1.0 weight% based on the polymer. The polymer was then milled into films for ten minutes at about 160°C (320°F) using hot rollers.

Films were then exposed in the weatherometer as in the previous test, for 100 and 300 of exposure.

Unexposed film, and film which had been exposed to the weather simulation for 100 and 300, respectively, were tested for antimicrobial activity as follows;

a) Samples of film which had been unexposed, exposed for 100 h and exposed for 300 were placed on samples of nutrient agar variously inoculated with bacteria, actinomycetes or fungi. The organisms were:

| | |
|---|---|
| *BACTERIA:* | |
| *Staphylococcus aureus* | ATCC 6538 (gram-positive) |
| *Klebsiella pneumoniae* | ATCC 4352 |

| | |
|---|---|
| *ACTINOMYCETES* | |
| *Stv. reticulum* | ATCC 25607 (pink staining organism) |

*FUNGI:* a mixed fungal spore suspension of:

| | |
|---|---|
| *Aspergillus niger* | ATCC 9642 |
| *Aspergillus flavus* | ATCC 9643 |
| *Penicillium funiculosum* | ATCC 9644 |
| *Chaetonium globosum* | ATCC 6205 |

The samples inoculated with bacteria or actinomycetes were incubated for 24 h at 37°C; those containing fungi were incubated for 14 d at 28°C. After incubation, activity was evaluated by measuring the size of a clear zone of no growth (i:e., zone of inhibition) around each sample and visually rating the degrees of fungal growth or stain on the sample.

b) A second series of fungicidal tests was conducted using similar samples placed on agar containing non-nutrient mineral salts and inoculated with a mixed fungal spore suspension of: *Aspergillus niger,* ATCC 9642, *Penicillin funiculosum,* ATCC 9644, *Chaetomium globosum,* ATCC 6205, *Aureobasidium pullulans,* ATCC 9348, and *Trichoderma sp.,* ATCC 9645. The samples were incubated for 21 d at 28°C (ASTM Standard method G. 21—75). Antifungal activity was evaluated by visually rating the degree of fungal growth on the samples.

The results of these tests are contained in the following Table III.

The samples were also visually examined for coloration, prior to exposure and after 100 h of exposure. The results are contained in the following Table IV.

TABLE III

| Com. No. | Stabi-lized | Wt.-% | Exposure (h) | Zone of Inhibition, mm/growth or staining: Staph. aureus | K. pneum. | Stv. ret. | Mixed fungi (a) | Mixed fungi (b)1 |
|---|---|---|---|---|---|---|---|---|
| 1 | – | 0.5 | 0 | 11/N | 3/N | 7/NS | 2/N | N |
| | – | 0.5 | 100 | 10/N | 2/N | 5/NS | 0—2/N | N |
| | – | 0.5 | 300 | 7/N | 1/N | 4/NS | 0/N | T |
| | + | 0.5 | 0 | 11/N | 3/N | 6/NS | 3/N | N |
| | + | 0.5 | 100 | 10/N | 2.5N | 5/NS | 2/N | N |
| | + | 0.5 | 300 | 9/N | 1.5/N | 5/NS | 0/N | N |
| | – | 1.0 | 0 | 15/N | 6.5/N | 10/NS | 4/N | N |
| | – | 1.0 | 100 | 13/N | 5/N | 7/NS | 3/N | N |
| | – | 1.0 | 300 | 10/N | 4/N | 6/NS | 2/N | N |
| | + | 1.0 | 0 | 15/N | 6/N | 9/NS | 5/N | N |
| | + | 1.0 | 100 | 13/N | 5/N | 7/NS | 4/N | N |
| | + | 1.0 | 300 | 10/N | 4/N | 7/NS | 2/N | N |
| 2 | + | 0.5 | 0 | 11/N | 3/N | 6/NS | 3/N | N |
| | + | 0.5 | 100 | 10/N | 2.5/N | 5/NS | 2/N | N |
| | + | 0.5 | 300 | 9/N | 1.5/N | 5/NS | 2/N | N |
| | + | 1.0 | 100 | 10/N | 3/N | 8.5/NS | 0—1/NS | N |
| | + | 1.0 | 100 | 10/N | 2/N | 6/NS | 0/N | N |
| | + | 1.0 | 300 | 6/N | 1.5/N | 6/NS | 0/N | T |
| 3 | + | 0.5 | 0 | 4/N | 0/G | 3.5/NS | 0/T | L |
| | + | 0.5 | 100 | 4/N | 0/G | 2/NS | 0/T | L |
| | + | 0.5 | 300 | 3.5/N | — | 2.5/NS | 0/T | L |
| | + | 1.0 | 0 | 5/N | 0/G | 5/NS | 0/T | T |
| | + | 1.0 | 100 | 5/N | 0/G | 3/NS | 0/T | T |
| | + | 1.0 | 300 | 5/N | — | 4/NS | 0/T | T |
| 4 | – | 0.5 | 0 | 9/N | 6/N | 9/NS | 0/N | N |
| | – | 0.5 | 100 | 8/N | 2.5/N | 6/NS | 0/T | N |
| | – | 0.5 | 300 | 5/N | 1/N | 5/NS | 0/T | T |
| | + | 0.5 | 0 | 11/N | 6/N | 9/NS | 0/N | N |
| | + | 0.5 | 100 | 9/N | 4/N | 7/NS | 0/N | N |

TABLE III (continued)

| Com. No. | Stabi-lized | Wt.-% | Exposure (h) | Zone of Inhibition, mm/growth or staining: Staph. aureus | K. pneum. | Stv. ret. | Mixed fungi (a) | Mixed fungi (b)1 |
|---|---|---|---|---|---|---|---|---|
| 4 | + | 0.5 | 300 | 5/N | 1/N | 5/NS | 0/N | T |
| | − | 1.0 | 0 | 12/N | 6/N | 9/NS | 0/N | N |
| | − | 1.0 | 100 | 10/N | 5/N | 6/NS | 0/T | N |
| | − | 1.0 | 300 | 8/N | 2/N | 4/NS | 0/T | T |
| | + | 1.0 | 0 | 12/N | 8/N | 10/NS | 0/N | N |
| | + | 1.0 | 100 | 11/N | 6/N | 7/NS | 0/N | N |
| | + | 1.0 | 300 | 8/N | 3/N | 4/NS | 0/N | N |
| 5 | − | 0.5 | 0 | 14/N | 9/N | 10/NS | 1/N | N |
| | − | 0.5 | 100 | 13/N | 8/N | 10/NS | 0/N | N |
| | − | 0.5 | 300 | 10/N | 4/N | 8/NS | 0/N | N |
| | + | 0.5 | 0 | 13/N | 10/N | 11/NS | 0—1/N | N |
| | + | 0.5 | 100 | 13/N | 9/N | 9/NS | 0—1/N | N |
| | + | 0.5 | 300 | 9/N | 4/N | 4/NS | 0—1/N | N |
| | − | 1.0 | 0 | 19/N | 13/N | 14/NS | 8/N | N |
| | − | 1.0 | 100 | 14/N | 10/N | 10/NS | 5/N | N |
| | − | 1.0 | 300 | 12/N | 6/N | 10/NS | 4/N | N |
| | + | 1.0 | 0 | 19/N | 13/N | 17/NS | 9/N | N |
| | + | 1.0 | 100 | 16/N | 12/N | 12/NS | 4/N | N |
| | + | 1.0 | 300 | 12/N | 7/N | 9/NS | 3/N | N |
| | + | 0.5 | 0 | 13/N | 10/N | 11/NS | 0—1/N | N |
| | + | 0.5 | 100 | 13/N | 9/N | 9/NS | 0—1/N | N |
| | + | 0.5 | 300 | 9/N | 4/N | 4/NS | 0—1/N | N |
| | − | 1.0 | 0 | 19/N | 13/N | 14/NS | 8/N | N |
| | − | 1.0 | 100 | 14/N | 10/N | 10/NS | 5/N | N |
| | − | 1.0 | 300 | 12/N | 6/N | 10/NS | 4/N | N |
| | + | 1.0 | 0 | 19/N | 13/N | 17/NS | 9/N | N |
| | + | 1.0 | 100 | 16/N | 12/N | 12/NS | 4/N | N |
| | + | 1.0 | 300 | 12/N | 7/N | 9/NS | 3/N | N |
| 6 | + | 0.5 | 0 | 9/N | 2/N | 4/NS | 0—1/N | N |

TABLE III (continued)

| Com. No. | Stabi-lized | Wt.-% | Exposure (h) | Zone of Inhibition, mm/growth or staining | | | | |
|---|---|---|---|---|---|---|---|---|
| | | | | Staph. aureus | K. pneum. | Stv. ret. | Mixed fungi (a) | Mixed fungi (b)1 |
| 6 | + | 0.5 | 100 | 8/N | 2/N | 4/NS | 0—1/N | N |
| | + | 0.5 | 300 | 6/N | 1/N | 4/NS | 0—1/N | N |
| | + | 1.0 | 0 | 12/N | 3/N | 11/NS | 3/N | N |
| | + | 1.0 | 100 | 9.5/N | 2.5/N | 8/NS | 3/N | N |
| | + | 1.0 | 300 | 7/N | 1/N | 7/NS | 3/N | N |
| 7 | + | 0.5 | 0 | 12/N | 6/N | 12/NS | 0/N | N |
| | + | 0.5 | 100 | 11/N | 5.5/N | 10/NS | 0/N | N |
| | + | 0.5 | 300 | 8/N | 2/N | 5/NS | 0/N | N |
| | – | 0.5 | 0 | 12/N | 6/N | 12/NS | 0/N | N |
| | – | 0.5 | 100 | 10/N | 4.5/N | 9/NS | 0/T | N |
| | – | 0.5 | 300 | 9/N | 2/N | 7/NS | 0/T | N |
| | + | 1.0 | 0 | 12/N | 8.5/N | 13/NS | 2/N | N |
| | + | 1.0 | 100 | 14/N | 8.5/N | 12/NS | 2/N | N |
| | + | 1.0 | 300 | 9/N | 5/N | 7/NS | 1/N | N |
| | – | 1.0 | 0 | 14/N | 8/N | 12/NS | 2/N | N |
| | – | 1.0 | 100 | 12/N | 7/N | 9/NS | 1/N | N |
| | – | 1.0 | 300 | 11/N | 4/N | 8/NS | 1/N | N |
| Control | + | 0 | 0 | 0/G | 0/G | 0/NS | 0/HG | HG |
| | + | 0 | 100 | O/G | 0/G | 0/NS | 0/HG | HG |
| | + | 0 | 300 | O/G | 0/G | 0/NS | 0/HG | HG |
| | – | 0 | 0 | O/G | 0/G | 0/NS | 0/HG | HG |
| | – | 0 | 100 | O/G | 0/G | 0/NS | 0/HG | HG |
| | – | 0 | 300 | O/G | 0/G | 0/TS | 0/HG | HG |

KEY TO TABLE III

Key: N — No growth in contact area
T — Trace growth in contact area (less than 10%)
L — Light growth in contact area (10—30%)
G — Growth in contact area (30—60%)
HG — Heavy growth in contact area (>60%)
NS — No staining in contact area
TS — Trace of staining in contact area
+ — U.V. stabilizer included
– — no U.V. stabilizer

TABLE IV

| Compound | Wt.-% | U.V. Stabilized | Color: No exposure | Color: 100 h exposure |
|---|---|---|---|---|
| 1 | 0.5 | – | C | C |
| | 1.0 | – | C | C |
| | 0.5 | + | C | C |
| | 1.0 | + | C | C |
| 2 | 0.5 | + | C | C |
| | 1.0 | + | C | C |
| 3 | 0.5 | + | T | Lt.T |
| | 1.0 | + | DK. T | Lt. T |

Key: + — U.V. Stabilizer included
– — U.V. Stabilizer not included
C — Color less
T — Tan
Lt. — Light
Dk. — Dark

Thus, formulations including Compounds 1 and 2 were highly stable toward ultraviolet radiation, as demonstrated by the fact that films containing these compounds did not become colored after exposure to ultraviolet light for 100 h. With respect to Compound 1, stability towards such radiation existed even in the absence of an ultraviolet stabilizer in the formulation. Such stability towards ultraviolet radiation would not have been expected from the structure of these compounds. All the compounds contain an activated double bond, which would be expected to be oxidized in the presence of ultraviolet light and air.

D) Long-term Exposure Test:

Compound 1 was utilized in this test. A dry powdered blend of polyvinyl chloride was prepared by dry mixing of the following ingredients (by weight): 100 parts Diamond Shamrock 450® polyvinyl chloride resin, 40 parts dioctyl phthalate plasticizer, 3.50 parts Mark KCB® barium-cadmium-zinc heat stabilizer/lubricant, 1.5 parts Mark C® organic phosphite heat stabilizer/lubricant, 1.0 part Mark 202A® benzophenone ultraviolet stabilizer, 0.25 parts stearic acid and 7.6 parts epoxidized soybean oil (plasticizer/stabilizer). Compound 1 was added to samples of this blend in amounts so as to provide compositions containing 0.065, 0.25 and 1.0% by weight of this compound. The compositions were further mixed for ten minutes, then milled into a film on hot rollers at 160°C (320°F).

Samples of the films thus produced were placed out-of-doors at a location in Puerto Rico and exposed to the weather for 8—12 months, continuously. Visual evaluations of microbial growth on the surface of the films were made using a scale of from 0 to 5, with 0 representing no growth visible to the naxed eye and 5 representing complete coverage. Results of these observations were as follows:

| Wt.-% Compound 1 | 8 months | 12 months |
|---|---|---|
| 0 (control) | 4 | 5 |
| 0.065 | <1 | 4 |
| 0.25 | 0 | 4 |
| 1.0 | 0 | 0 |

Comparative Activity Tests:
Commercial Agricultural and Soil Fungicides

As U.S. patent 3,159,532 indicates, the compounds in question have utility as soil fungicides. Several commercial agricultural and soil fungicides were tested by the above-described methods to ascertain whether any correlation could be determined between activity as a soil fungicide and activity as a biocide

for plastics or polymers. The tests appeared to demonstrate a lack of any such correlation. In particular:

Cycloheximide, 3-[2-(3,5-dimethyl-2-oxacyclohexyl)-2-hydroxyethyl]glutaramide, tested as in (B) above, at 0.5 wt.-%, provided essentially no control of the test organisms in the contact area, nor any zone of inhibition, both before and after exposure.

Thiram, bis(dimethylthio-carbamoyl) disulfide, tested as in (B) at levels of 0.5—2.0 wt.-%, provided essentially no control of the test organisms in the contact area with the exception of the mixed fungal spore, and no zone of inhibition, with the exception of the mixed fungal spore at 2.0 wt.-%, both before and after exposure.

Captafol, cis-N-[(1,1,2,2-tetrachloroethyl)thio]-4-cyclohexene-1,2-dicarboximide, tested as in (C) at 0.5 and 0.75 wt.-%, with up to 300 h exposure, controlled test organisms as follows: *Staph. aureus* — 2.5—3.5 mm inhibition/N; *K. pneumoniae* — 0.5—2 mm inhibition/N; *Stv. reticulum* — 4—6 mm inhibition/NS; mixed fungi — 0.3 mm inhibition/N (with large zone of inhibition of *Aspergillus niger).*

Captan, cis-N-[(trichloromethyl)thio]-cyclohexene-1,2-dicarboximide, tested as in (C), at 0.5—1.05 wt.-%, controlled test organisms as follows: *Staph. aureus* — 0.5—3.5 mm inhibition/N; *K. pneumoniae* — 0—1 mm inhibition with no growth in the contact area after 200 h of exposure, but growth occurring after 300 h; *Stv. reticulum* — 0.5—4 mm inhibition/NS; mixed fungal spore — 0—5 mm inhibition/N. Inhibition of fungal growth dropped sharply with exposure.

Captan, however, caused yellowing of polyvinyl chloride films after exposure. Unweathered films containing Captan at greater than 0.5 wt.-% exhibited surface blooming, that is, formation of white powder on the surface, believed to be Captan. No powder was seen on the surfaces of weather-exposed films; however such may have been washed off during the exposure treatment.

Methods of Formulations:

For use as plastics or polymer biocides, the compounds disclosed herein may be incorporated in plastics or films or products made therefrom, in an amount ranging from 0.001 to 2.0% by weight of the total composition, preferably 0.01 to 1.0, and most preferably 0.01 to 0.5% by weight. The incorporation may be performed, as was done in the examples, by incorporating an amount of the biocide in a dry mix which is then processed to produce the desired plastic or polymer or ultimate product containing it. Alternatively, the biocide may be incorporated in a dry blend at a higher rate, for instance, 12—13% by weight, to which a small amount of an extrusion aid (for instance barium-calcium stearate) is added, and the blend extruded to form a rod, from which pellets are cut, as described for instance in U.S. Patent 4,086,297. The pellets can then be utilized as a means to incorporate the biocide into subsequent plastic or polymer formulations, with the number of pellets added being determined so as to produce an overall polymer composition including the biocide in the desired or appropriate amount.

More commonly, the antimicrobial compound is predissolved or dispersed in a liquid carrier solvent such as a plasticizer for a polymeric resin, see for instance U.S. Patent 3,288,674, thereby providing a vehicle for the biocide for ease of incorporation and to promote its migration throughout the resin, particularly to its surface. Usually, the biocide is dissolved in a first solvent and then diluted with the desired resin-compatible plasticizer second solvent to provide a final liquid solution wherein the first solvent acts as a coupling solvent for the biocide and plasticizer to maintain homogeneity. Both first and second solvents are themselves mutually compatible with each other and the polymeric resin system. See, for instance, U.S. Patent 3,288,674.

**Claims**

1. The use of a compound having the formula

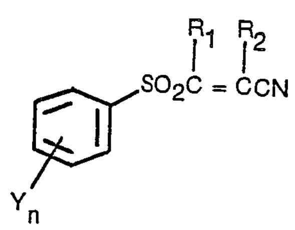

in which Y is hydrogen, halogen, $C_1$—$C_4$ alkoxy, or $C_1$—$C_4$ alkyl; $R_1$ and $R_2$ are independently hydrogen or methyl; and n is 1 or 2, as a microbiocidally active additive for the inclusion in synthetic polymer compositions.

2. A synthetic polymer composition and articles made therefrom, characterized by the inclusion of a compound having the formula

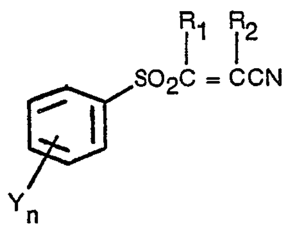

in which Y is hydrogen, halogen, $C_1$—$C_4$ alkoxy, or $C_1$—$C_4$ alkyl; $R_1$ and $R_2$ are independently hydrogen or methyl; and n is 1 or 2 in a microbiocidally effective amount.

3. A composition and an article made therefrom according to claim 2, characterized in that said composition comprises polyvinyl chloride.

4. A composition and an article made therefrom according to one of the claims 2 or 3, characterized in that said compound is included in an amount of between 0.001 and 2.0% by weight.

5. A dry blend and articles made therefrom for use as a microbiocidal additive for synthetic polymer compositions comprising a compound having the formula

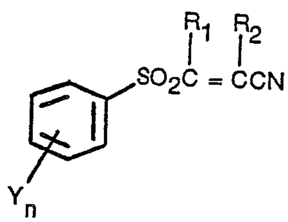

in which Y is hydrogen, halogen, $C_1$—$C_4$ alkoxy, or $C_1$—$C_4$ alkyl; $R_1$ and $R_2$ are independently hydrogen or methyl; and n is 1 or 2, in an amount of 12 to 13% by weight and an extrusion aid.

6. Pellets as a microbiocidal additive for synthetic polymer compositions made by extruding the dry blend of claim 5 to form a rod, from which the pellets are cut.

**Patentansprüche**

1. Verwendung einer Verbindung der Formel

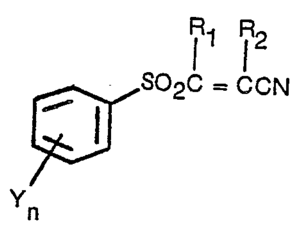

in der Y Wasserstoff, Halogen, $C_1$—$C_4$-Alkoxy oder $C_1$—$C_4$-Alkyl; $R_1$ und $R_2$ unabhängig voneinander Wasserstoff oder Methyl und n = 1 oder 2 sind, als ein mikrobiozid wirksames Additiv für den Einschluß in synthetischen polymeren Zusammensetzungen.

2. Eine synthetische polymere Zusammensetzung und daraus hergestellte Gegenstände, gekennzeichnet durch den Einschluß einer Verbindung gemäß der Formel

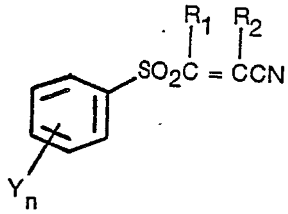

in einer mikrobiozid wirksamen Menge, wobei Y Wasserstoff, Halogen, $C_1$—$C_4$-Alkoxy oder $C_1$—$C_4$-Alkyl; $R_1$ und $R_2$ unabhängig voneinander Wasserstoff oder Methyl und n = 1 oder 2 sind.

3. Eine Zusammensetzung und ein daraus hergestellter Gegenstand gemäß Anspruch 2, dadurch gekennzeichnet, daß die Zusammensetzung Polyvinylchlorid enthält.

4. Eine Zusammensetzung und ein daraus hergestellter Gegenstand gemäß einem der Ansprüche 2 oder 3, dadurch gekennzeichnet, daß die Verbindung in einer Menge zwischen 0,001 und 2,0 Gew.-% eingeschlossen ist.

5. Eine trockene Mischung und daraus hergestellte Gegenstände zur Verwendung als mikrobiozid wirksames Additiv für synthetische polymere Zusammensetzungen, wobei eine Verbindung gemäß der Formel

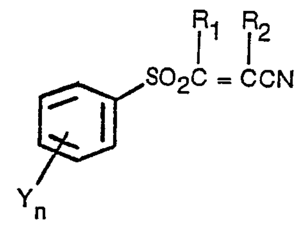

wobei Y Wasserstoff, Halogen, $C_1$—$C_4$-Alkoxy oder $C_1$—$C_4$-Alkyl; $R_1$ und $R_2$ unabhängig voneinander Wasserstoff oder Methyl und n = 1 oder 2 sind, in einer Menge von 12 bis 13 Gew.-% und ein Extrusionshilfsmittel enthalten sind.

6. Pellets als microbiozid wirksames Additiv für synthetische polymere Zusammensetzungen, die durch Extrusion der trockenen Mischung gemäß Anspruch 5 unter Bildung eines Stabes und durch Abschneiden der Pellets gebildet werden.

**Revendications**

1. L'application d'un composé présentant la formule:

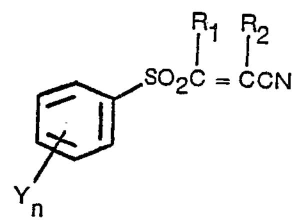

dans laquelle:

Y est un atome d'hydrogène, un halogène, un radical alkoxy en $C_1$ à $C_4$ ou alkyle en $C_1$—$C_4$,

$R_1$ et $R_2$ indépendamment l'un de l'autre sont un atome d'hydrogène ou le radical méthyle; et

n est égal à 1 ou 2,

en tant qu'adjuvant actif du point de vue microbiocide destiné à être inclus dans des compositions de polymère synthétique.

2. Composition de polymère synthétique et articles qui en dérivent, caractérisés par l'inclusion d'un composé présentant la formule:

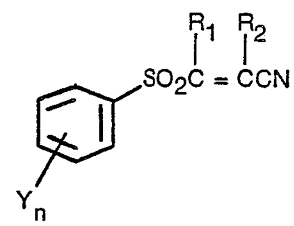

dans laquelle:

Y est un atome d'hydrogène ou d'halogène, un radical alkoxy en $C_1$ à $C_4$- ou alkyle en $C_1$ à $C_4$,

$R_1$ et $R_2$ indépendamment l'un de l'autre sont un atome d'hydrogène ou le radical méthyle; et

n est égal à 1 ou 2,

utilisé dans une quantité efficace du point de vue microbiocide.

3. Une composition et un article qui en dérive selon la revendication 2, caractérisés en ce que ladite composition comprend du chlorure de polyvinyle.

4. Une composition et un article qui en dérive selon l'une quelconque des revendications 2 ou 3, caractérisé que ce que ledit composé y est présent en quantité comprise entre 0,001 et 2,0% en poids.

5. Mélange sec et articles en dérivant destinés à être utilisés en tant qu'adjuvant microbiocide pour compositions polymères synthétiques comprenant un composé présentant la formule:

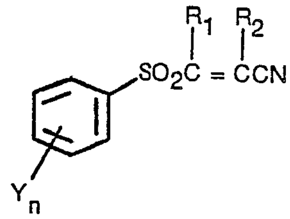

dans laquelle:

Y est un atome d'hydrogène ou d'halogène, un radical alkoxy en $C_1$ à $C_4$ ou alkyle en $C_1$ à $C_4$,

$R_1$ et $R_2$ indépendamment l'un de l'autre sont un atome d'hydrogène ou un radical méthyle; et

n est égal à 1 ou 2,

présent en quantité comprise entre 12 et 13% en poids, et un adjuvant d'extrusion.

6. Granulés constituant un additif microbiocide pour compositions polyméres synthétiques produits par extrusion du mélange sec de la revendication 5 pour former une tige, à partir de laquelle les granulés sont coupés.